# EUROPEAN PATENT APPLICATION

(11) **EP 0 542 468 A1**
(43) Date of publication of application: **19.05.1993**
(21) Application number: 92310097.8
(22) Date of filing: 04.11.1992
(51) Int. Cl.: C07D 275/02, C07D 275/04, A01N 43/80

(54) **Isothiazolyl compounds and their use as microbicides**

(30) Priority: 12.11.1991 US 791753
(71) Applicant: ROHM AND HAAS COMPANY, Philadelphia Pennsylvania 19105 (US)
(72) Inventor: Gironda, Kevin Fitzgerald, Alpha, New Jersey 08865 (US); Osei-Gyimah, Horsham, Pennsylvania 19044 (US); Lange, Barry Clifford, Lansdale, Pennsylvania 19446 (US)
(74) Representative: Smith, Julian Philip Howard

(57) **Abstract**

Compounds of the formula **I** 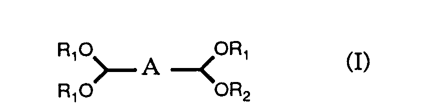
wherein
A is a C-C bond or any residue of a dialdehyde;
R₁ is independently selected from the group consisting of R₂ and (C₁-C₁₈)alkyl and;
R₂ is 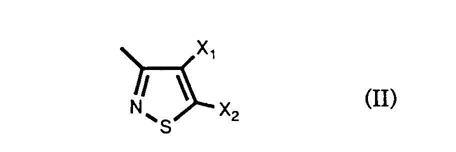
wherein
(i) X₁ and X₂ can be joined to form a 5 or 6 membered fused carbocyclic ring, said ring being saturated, unsaturated, or aromatic; or
(ii)
   X₁ = Cl, H, methyl, or Br; and
   X₂ = H, Cl, or Br.

## Description

This invention concerns antimicrobial compounds and compositions. In particular, it relates to new isothiazolyl compounds which are useful as microbicides and are referred to as girondalones.

Lewis et al., U.S.-A-3,761,488; 3,544,580; 3,835,150; 3,706,757; and 4,105,431, all assigned to Rohm and Haas Co., the same assignee as the present invention, disclose 3-isothiazolone compounds. Willingham et al. in U.S. Serial Nos. 438,816, filed November 17, 1989 and 601,964, filed October 22, 1990, both assigned to Rohm and Haas Company, disclose stabilizing said isothiazolone compounds with carbonyl compounds. Other stabilization methods for isothiazolones are disclosed in a series of patents assigned to the same assignee.

It has been a problem in the art to find an ideal stabilization method for the 3-isothiazolone compounds, which are outstanding antimicrobial compounds but which are chemically and thermally unstable unless one of the aforementioned stabilization systems is used. Another problem with the 3-isothiazolones is that they can be skin sensitizers under certain conditions.

In the search for a new stabilization method for 3-isothiazolone compounds, and also for ways to decrease the skin sensitization potential of the 3-isothiazolones, a new class of compounds was unexpectedly discovered.

Accordingly the present invention provides in one aspect a compound of the formula I 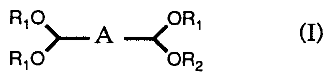
wherein
A is a C-C bond or any residue of a dialdehyde;
R₁ is the same as R₂ or_(C₁-C₁₈)alkyl ;
R₂ is 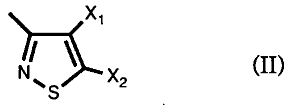
wherein
(i) X₁ and X₂ can be joined to form a 5 or 6 membered fused carbocyclic ring, said ring being saturated, unsaturated, or aromatic,
   or
(ii) X₁ = Cl, H, Me or Br, and X₂ = H, Cl, or Br.

By residue of a dialdehyde is meant any moiety which could have originated from a dialdehyde, the two CHO groups of the dialdehyde becoming (ultimately) CH(OR₁)₂ and CH(OR₂)₂ groups, and A being equivalent to the original dialdehyde minus the two CHO groups. Thus such a dialdehyde may be represented by the formula OHC-A-CHO. Examples of a suitable dialdehydes of which the residues are represented by A are glutaraldehyde; malonaldehyde; succinaldehyde; octanedial; 2,2-sulfonyl-bis-acetaldehyde; 2,2-oxy-bis-acetaldehyde; 2,2-alkylphosphine oxide; bis-acetaldehyde; succinic acid-2,3-bis-acetaldehyde; phthaldehyde; homophthalaldehyde; 4-octenedial; 2-cyclopentene-1,4-dial, and glyoxal. If the dialdehyde is glyoxal (OHC-CHO), the residue A is simply the C-C bond, in which case the compounds of formula (I) can be represented by formula (V) as follows:

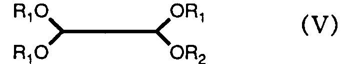

The invention also includes compositions comprising the above compounds and a pharmaceutically or agronomically acceptable carrier.

In another aspect the invention provides a method of preparing a compound of formula I comprising reacting a compound of the formula 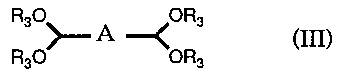
wherein R₃ is (C₁-C₁₈)alkyl,
with 3-hydroxyisothiazole or a substituted derivative thereof of the formula

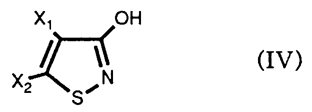

It is preferred to conduct the reaction by using heat and in the presence of a weak acid such as propionic acid. The product is preferably a mixture of compounds of formula I with some compounds having no R₁ = R₂, some having one R₁ = R₂, and some having two or three R₁ = R₂. The compound (III) can be made from a dialdehyde of the formula OHC-A-CHO by well-known means.

The invention also encompasses in a further aspect a method of preventing or inhibiting the growth of bacteria, fungi, algae or yeast in a locus susceptible or subject to contamination thereby, comprising introducing into or onto said locus a compound or composition as defined in any preceding claim in an amount effective to adversely affect said growth. The use of such compounds or compositions as microbicides is also a part of the invention.

The compounds of the invention, hereinafter referred to as girondalones, are especially active and efficient antimicrobial compounds.
Among the preferred compounds of formula I are the following:
1.) 1,3,3-trimethoxy-1-(5-chloro-isothiazoloxy-3yl) propane;
2.) 1,3-dimethoxy-1,3-bis-(5-chloro-isothiazoloxy-3-yl) propane;
3.) 1,3,3-trimethoxy-1-(isothiazoloxy-3-yl) propane; and
4.) 1,3-dimethoxy-1,3-bis-(isothiazoloxy-3-yl) propane

The melting point of compound 2 was 57°C. Compounds 1, 3 and 4 are oils.

The girondalone compounds are useful as antimicrobial compounds in a wide variey of applications. Generally they are suitable for the same applications as 3-isothiazolone compounds, whose uses are described in many of the prior art references cited above. The amounts of the girondalone compound to be used depend on the particular application. The useful amounts for a particular application are similar to amounts used for other microbicidal compounds. The girondalone compounds can be used in combination with other microbicides. The term "microbicide" is considered equivalent to "antimicrobial" as used herein.

Suitable methods of application of girondalone compounds of formula I to control fungi, bacteria, algae, viruses, yeasts, and the like are in amounts and with carriers, etc., as are well known in the art.

The following examples are presented to illustrate a few embodiments of the invention, but are not to be considered as limiting.

### Example 1

1,3,3-Trimethoxy-1-(isothiazoloxy-3-yl) propane and 1,3-Dimethoxy-1,3-bis-(isothiazoloxy-3-yl) propane

To a stirred mixture of 3-hydroxy-isothiazole (6g, 0.0594m) and malonaldehyde-bis(dimethyl acetal) (8.24g, 0.0502m), propionic acid (0.2 g, 0.0027m) was added.

The mixture was heated at 100°C with stirring for 14 hours, cooled, and poured into saturated sodium bicarbonate solution. The mixture was extracted with ethyl acetate. The ethyl acetate portion was washed with water, dried (MgSO₄), and concentrated to give a mixture of 1,3,3-trimethoxy-1-(isothiazoloxy-3-yl) propane and 1,3-dimethoxy-1,3-bis-(isothiazoloxy-3-yl) propane as an oil.

The girondalone compounds were separated by column chromatography on silica gel by first eluting with ethyl acetate to give 2.7 g 1,3,3-trimethoxy-1-(isothiazoloxy-3-yl) propane followed by methanol to elute 1,3-dimethoxy-1,3-bis-(isothiazoloxy-3-yl) propane (2.5 g.).
- NMR (d₆-acetone): 8.6(1H), 6.2(1H), 5.7(1H), 4.5(1H), 3.3(9H), 2.0 (m , 2H)
8.7(2H), 6.3(2H), 5.7(1H), 5.4(1H), 3.3(6H), 2.0-2.7 (m , 2H).

### Example 2: 1,3,3-Trimethoxy-1-(5-chloro-isothiazoloxy-3-yl) propane and 1,3-Dimethoxy-1,3-bis-(5-chloro-isothiazoloxy-3-yl) propane

To a stirred mixture of 5-chloro-3-hydroxy-isothiazole (2g, 0.0148m) and malonaldehyde bis(dimethyl acetal) (3.42g, 0.0209m), propionic acid (0.12g, 0.0020m) was added. The mixture was heated at 100°C. with stirring for 12 hours, then cooled and poured into saturated sodium bicarbonate solution. The mixture was extracted with ethyl acetate. The ethyl acetate portion was washedwith water, dried (MgSO₄), and concentrated. The girondalone products were separated by column chromatography using ethyl acetate:hexane (1:1) as eluant to yield 0.44 g. of 1,3,3-trimethoxy-1-(5-chloro-isothiazoloxy-3-yl)propane as an oil and 0.80g of 1,3-dimethoxy-1,3-bis-(5-chloro-isothiazoloxy-3-yl) propane as a solid; mp 57°C.
- NMR (d₃-chloroform): 6.3(1H), 5.8(1H), 4.6(1H), 3.4(9H), 2.1(m ,2H)
7.4(1H), 6.4(1H), 5.9(1H), 5.7(1H), 3.5(6H), 2.4 (m ,2H).

### Example 3

### Biological Activity

Efficacy against bacteria and fungi was carried out. A minimum inhibitory concentration (MIC) value was obtained using Trypticase Soy Broth (TSB) or a complex nutrient broth known as M9G, pH 7.0 and preparing a serial dilution with a starting concentration of 500 ppm. A stock solution of the test compound was made in dimethyl sulfoxide/acetone/water mixture. The test organisms used to demonstrate biocidal activity are listed in Table 1. The MIC's of the compounds of this invention against the test organisms are shown in Table 2. The tests were repeated with the media being adjusted to pH 5, 7, and 8.5. The results are shown in Tables 3 and 4. The following girondalones were evaluated:
1.) 1,3,3-trimethoxy-1-(5-chloro-isothiazoloxy-3 yl) propane;
2.) 1,3-dimethoxy-1,3-bis-(5-chloro-isothiazoloxy-3-yl) propane;
3.) 1,3,3-trimethoxy-1-(isothiazoloxy-3-yl) propane; and
4.) 1,3-dimethoxy-1,3-bis-(isothiazoloxy-3-yl) propane

| TABLE 1: Microorganisms used in the Antimicrobial Test | | |
|---|---|---|
| | Name | Abbreviations Used |
| **Bacteria** | Pseudomonas aeruginosa | Psae |
| | Escherichia coli | Ecol |
| | Staphlococcus aureus | Saur |
| **Fungi** | Aspergillus niger | Anig |

| TABLE 2: MIC Values (um) | | | | | | | |
|---|---|---|---|---|---|---|---|
| | Psae | | Ecol | | Saur | | Anig |
| Compound | M9G | TSB | M9G | TSB | TSB | TSB | TSB |
| 1 | 0.11 | 0.22 | 0.01 | 0.06 | <.0.001 | 0.011 | 0.01 |
| 2 | 0.08 | 0.16 | 0.04 | 0.08 | <0.001 | 0.01 | 0.02 |
| 3 | 0.50 | 0.25 | 0.13 | 0.13 | 0.06 | 0.06 | 0.50 |
| 4 | 0.37 | 0.05 | 0.10 | 0.19 | 0.01 | 0.02 | 0.75 |

| TABLE 3: MINIMUM INHIBITORY CONCENTRATION (um) | | | | | | |
|---|---|---|---|---|---|---|
| Compound | Pseudomonas aeruginosa | | | Staphylococcus aureus | | |
| **pH:** | 5 | 7 | 8.5 | 5 | 7 | 8 |
| 1 | 0.23 | 0.44 | 0.44 | 0.11 | 0.23 | 0.44 |
| 2 | 0.08 | 0.31 | 0.31 | 0.02 | 0.16 | 0.31 |

| TABLE 4: MINIMUM INHIBITORY CONCENTRATION(um) | | | | | | |
|---|---|---|---|---|---|---|
| Compound | Pseudomonas aeruginosa | | | Staphylococcus aureus | | |
| **pH:** | 5 | 7 | 8.5 | 5 | 7 | 8.5 |
| 3 | 1.00 | 2.01 | 2.01 | 0.13 | 0.50 | 1.00 |
| 4 | 1.50 | >1.50 | >1.50 | 0.19 | 0.37 | 0.10 |

Although specific embodiments and examples have been described herein, these have been provided by way of explanation and illustration and that the present invention is not limited thereby. Modifications which are within the ordinary skill in the art to make are considered to lie within the scope of the invention.

## Claims

1. Compound of the formula I 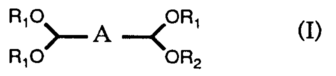 wherein
A is a C-C bond or any residue of a dialdehyde;
R₁ is the same as R₂ or_(C₁-C₁₈)alkyl ;
R₂ is 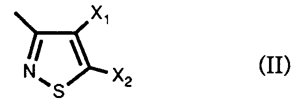 wherein
(i) X₁ and X₂ can be joined to form a 5 or 6 membered fused carbocyclic ring, said ring being saturated, unsaturated, or aromatic;
or
(ii)
X₁ = Cl, H, methyl, or Br; and
X₂ = H, Cl, or Br.

2. Compound according to claim 1 wherein A is a residue of glutaraldehyde; malonaldehyde; succinaldehyde; octanedial; 2,2-sulfonyl-bis-acetaldehyde; 2,2-oxy-bis-acetaldehyde; 2,2-alkylphosphine oxide bis-acetaldehyde; succinic acid-2,3-bis-acetaldehyde; phthaldehyde; homophthalaldehyde; 4-octenedial; 2-cyclopentene-1,4-dial; or glyoxal.

3. Compound according to claim 1 or 2 wherein said compound is 1,3,3-trimethoxy-1-(5-chloro-isothiazoloxy-3-yl)propane; 1,3-dimethoxy-1,3-bis-(5-chloro-isothiazoloxy-3-yl)propane;1,3,3-trimethoxy-1-(isothiazoloxy-3-yl)propane; or 1,3-dimethoxy-1,3-bis-(isothiazoloxy-3-yl) propane.

4. Microbicidal composition comprising a compound according to any preceding claim, and a pharmaceutically or agronomically acceptable diluent or carrier.

5. Method of preventing or inhibiting the growth of bacteria, fungi, algae or yeast in a locus susceptible or subject to contamination thereby, comprising introducing into or onto said locus a compound or composition as defined in any preceding claim in an amount effective to adversely affect said growth.

6. Process for preparing compounds as defined in any of claims 1 to 3 comprising reacting a compound of the formula 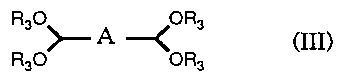 wherein R₃ is (C₁-C₁₈) alkyl
with 3-hydroxyisothiazole or a substituted derivative thereof the formula 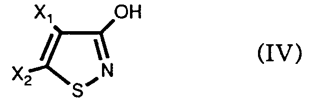 wherein X₁ and X₂ are as defined in claim 1.

7. Process according to claim 6 wherein said reaction is conducted in the presence of weak acid at elevated temperatures.

8. Process according to claim 6 or 7 wherein the compound of formula (III) is made from a dialdehyde of formula OHC-A-CHO.

9. Use of a compound or composition as defined in any of claims 1 to 4 as a microbicide.
